# EUROPEAN PATENT APPLICATION

(11) **EP 1 302 179 A2**
(43) Date of publication of application: **16.04.2003**
(21) Application number: 02257085.7
(22) Date of filing: 11.10.2002
(51) Int. Cl.: A61F 2/06

(54) **Flexible stent**

(30) Priority: 16.10.2001 US 981245
(71) Applicant: Cordis Corporation, Miami Lakes Florida 33014 (US)
(72) Inventor: Luehrs, Kirsten Freislinger, Palo Alto, CA 94306 (US)
(74) Representative: Belcher, Simon James

(57) **Abstract**

A flexible stent (10) incorporates struts (30,40) which define diamond shapes (20), in which the stiffness of the struts defining the diamond shapes varies to provide control over the flexing of the stent. Preferably, a diamond shape is defined by long stiff struts (30) and shorter struts (40) which are more flexible.

## Description

The present invention generally relates to medical devices, particularly stents and covered stents. More particularly, the present invention is directed to a stent which allows for recapturing and which possesses unusual flexibility.

As background to a discussion of stents, one notes that in the 1970s, the technique of percutaneous transluminal coronary angioplasty (PTCA) was developed for the treatment of atherosclerosis. Atherosclerosis is the build-up of fatty deposits or plaque on the inner walls of a patient's arteries; these lesions decrease the effective size of the artery lumen and limit blood flow through the artery, prospectively causing a myocardial infarction or heart attack if the lesions occur in coronary arteries that supply oxygenated blood to the heart muscles. In the angioplasty procedure, a guide wire is inserted into the femoral artery and is passed through the aorta into the diseased coronary artery. A catheter having a balloon attached to its distal end is advanced along the guide wire to a point where the sclerotic lesions limit blood flow through the coronary artery. The balloon is then inflated, compressing the lesions radially outward against the wall of the artery and substantially increasing the size of its internal lumen, to improve blood circulation through the artery.

Stents are increasingly being used in place of or in addition to PTCA for treatment of atherosclerosis, with the intent of minimizing the need to repeatedly open an atherosclerotic artery. Although a number of different designs for stents have been published, stents are generally configured as elongate cylindrical structures that are provided in a first state and can assume a second, different state, with the second state having a substantially greater diameter than the first state. A stent is implanted in a patient using an appropriate delivery system for the type of stent being placed within the patient's arterial system. There are two basic types of stents--those that are expanded radially outward due to the force from an inflated angioplasty type balloon, such as the Palmaz-Schatz stent, the Gianturco-Roubin stent and the Strecker stent, and those that are self expanding, such as the Maass double helix spiral stent, the Nitinol stent (made of nickel titanium memory alloy), the Gianturco stent and the Wallstent.

Stents may be used in combination with a PTCA procedure. Specifically, stents are sometimes used following a PTCA procedure if the artery is totally occluded or if the lesions have occluded a previously placed surgical graft. Typically, a stent constrained within an introducer sheath is advanced to a site within the patient's artery through a guide catheter. For the balloon-expanded type, after the introducer sheath is retracted, a balloon disposed inside the stent is inflated to a pressure ranging from about six to ten atmospheres. The force produced by the inflated balloon expands the stent radially outward beyond its elastic limit, stretching the vessel and compressing the lesion to the inner wall of the vessel.

A self expanding stent expands due to spring force following its emplacement in the artery, after a restraining sheath is retracted from the compressed stent, or in the case of the Nitinol version, the stent may sometimes assume its expanded memory state after being warmed above the transition temperature of the Nitinol alloy (e.g., above 30 degrees C).

Following the expansion process, when the balloon catheter is used, the balloon is removed from inside the stent and the catheter and other delivery apparatus is withdrawn. The lumen through the vessel is then substantially increased, improving blood flow. After a stent or other endoluminal device is implanted, a clinical examination and either an angiography or an ultrasonic morphological procedure is performed to evaluate the success of the stent emplacement procedure in opening the diseased artery or vessel. These tests are typically repeated periodically, e.g., at six-month intervals, since restenosis of the artery may occur.

Implantable devices may be used in other contexts, such as for abdominal aortic aneurysms. The abdominal aortic aneurysm usually arises in the infrarenal portion of the diseased aorta, for example, below the kidneys. When left untreated, the aneurysm may eventually cause rupture of the sac with ensuing fatal haemorrhaging in a very short time. High mortality associated with the rupture led initially to transabdominal surgical repair of abdominal aortic aneurysms. Surgery involving the abdominal wall, however, is a major undertaking with associated high risks. There is considerable mortality and morbidity associated with this magnitude of surgical intervention, which in essence involves replacing the diseased and aneurysmal segment of blood vessel with a prosthetic device, which typically is a synthetic tube, or graft, usually fabricated of polyester, Urethane, or from materials such as those sold under the trade marks DACRON and TEFLON.

Generally, stents, grafts, and graft stents are implantable medical devices (sometimes termed implantable tubular prostheses) that are placed within blood vessels and other body passageways to treat disease conditions such as stenoses, occlusions, and aneurysms. Transluminal implantation of such devices requires that they be introduced to the site collapsed about or within an introduction device and released to self expand or are expanded by other mechanisms to an expanded tubular state providing a lumen of approximately the same size as the patent vessel or duct lumen.

When the body lumen is weakened, for example, dissectional artery lining occurs in a body lumen such as a blood vessel, the weak part of the body lumen might collapse to occlude a fluid passageway. To prevent such an occlusion, a stent is implanted within the blood vessel to support the blood vessel from the inside thereof. Namely, a stent is delivered to a desired location in the blood vessel, and expanded in a circumferential direction in the blood vessel to support and maintain the patency of the blood vessel. Using the stent to support the blood vessel can avoid surgical exposing, incising, removing, replacing or bypassing a defective blood vessel required in the conventional vascular surgery.

Stents can be viewed as scaffoldings, of generally cylindrical symmetry, that function to physically support, and, if desired, expand the wall of the passageway. Typically, a stent consists of two or more struts or wire support members connected together into a lattice-like or open weave frame. Most stents are compressible for insertion through small cavities, and are delivered to the desired implantation site percutaneously via a catheter or similar transluminal device. Once at the treatment site, the compressed stent is expanded to fit within or expand the lumen of the passageway. Stents are typically either self-expanding or are expanded by inflating a balloon that is positioned inside the compressed stent at the end of the catheter. Intravascular stents are often deployed after coronary angioplasty procedures to reduce complications, such as the collapse of arterial lining, associated with the procedure.

There have been introduced various types of stents, and they can be typically categorized from viewpoints of methods for expanding the stent, shapes, methods for manufacturing the stent, designs and so on. From a viewpoint of methods for expanding the stent, stents can be categorized as a self-expandable stent, which can be expanded by itself, and a balloon expandable stent. In the balloon expandable stent, the stent is mounted on an expandable member, such as a balloon, provided on a distal end of an intravascular catheter, and the catheter is advanced to the desired location in the body lumen to deliver the stent. Then, the balloon on the catheter is inflated to expand the stent into a permanent expanded condition, and the balloon is deflated for removing the catheter from the stent.

From a viewpoint of the materials, stents can be categorized into a tubular stent and a tubular coil, and from a viewpoint of methods for manufacturing the stent, stents can be categorized as an etched stent and a laser cut stent. Then, from a viewpoint of designs, stents can be categorized into numerous types, but roughly, stents can be categorized into a stent having a zigzag pattern on the surface thereof, and a stent having a diamond pattern on the surface thereof.

A variety of expandable intraluminal vascular grafts are disclosed in US-4733665, US-4739762, US-4776337 US-5102417. The '665 document discloses stents that are expanded using angioplasty balloons. The stents are variously a wire mesh tube or of a plurality of thin bars fixedly secured to each other. The devices are installed, e.g., using an angioplasty balloon and consequently are not seen to be self-expanding. The'762 and '337 documents disclose stents which include thin-walled, biologically inert materials on their outer peripheries. The '417 document discloses the use of multiple stent sections each flexibly connected to its neighbour.

In all types of stents, the stent expands from an initial diameter to a larger diameter so as to be suitable for a particular size of the targeted body cavity. Therefore, the stent must have expandability in the circumferential direction. Also, since the stent is placed in the body lumen is to support a cavity wall therein to maintain the patency thereof, it is very important that the stent has radial strength as well as support capability.

At the same time, since the stent is generally delivered through tortuous path to the desired location in the body lumen, the stent must have flexibility in the axial direction. Namely, the stent must be flexible and is bent easily to thereby facilitate the delivery of the stent in the narrow and meandering body lumen.

Since simply bending a flexible wire makes a wire stent, the wire stent is not only expanded easily, but also shrunk easily. Namely, the wire stent does not have support capability for maintaining the expanded condition in order to keep the body lumen open. On the other hand, a tube type stent has enough support capability to maintain its expanded condition for holding the body lumen open.

In the zigzag pattern stent such as the Gianturco Z stent, struts attached in zigzag shape are connected in the circumferential direction to form a tubular shape stent. Therefore, when the stent is expanded, the zigzag shaped struts expand in the circumferential direction, but easily pushed back. Although the zigzag pattern stent has flexibility in the axial direction to facilitate the delivery of the stent in the body lumen, the zigzag pattern stent does not have enough radial strength and support capability to support the body lumen and maintain the patency thereof.

In a stent having a diamond pattern, struts forming diamond-like shapes are connected in the circumferential direction to form a tubular shape stent. As the diamond pattern stent opens, the diamond shapes expand to give scuffling structure to the stent for excellent radial strength to maintain the patency of the body lumen.

Accordingly, an object of the invention is to provide a stent, which has a high degree of flexibility in the axial direction to advance through narrow tortuous passageways.

Another object of the invention is to provide a stent as stated above, which also has strong radial strength and support capability in the circumferential direction.

Another object of the present invention is to make a kink resistant stent.

Specifically, an embodiment of the present invention pertains to a stent design which provides flexibility through an unstable, buckling diamond network, thus providing flexibility without drawbacks in terms of recapturability, coverage and/or delivery profile.

An embodiment of the present invention using a diamond-like pattern where the expanded shape is oversized to create an extremely strong structure that will resist flattening from external loads, such as those required in "kissing" configuration.

All of the stents disclosed herein are intended to have graft material attached thereto by using conventional techniques.

The present invention is generally directed to a stent design, which incorporates a diamond pattern.

In one embodiment, a flexible stent is designed by taking a diamond shaped design cutting it out of Nitinol and over-expanding it. This over expansion causes the individual struts to preferentially bend circumferentially. Ordinarily, they would preferentially bend longitudinally, facilitating pulsatile motion and diametric change. The reduction of strut thickness and the incorporation of a pattern of rigid or regularly expanded members facilitate the utility of this type of geometry. A strut oriented at approximately 45 degrees will allow bending both circumferentially and longitudinally.

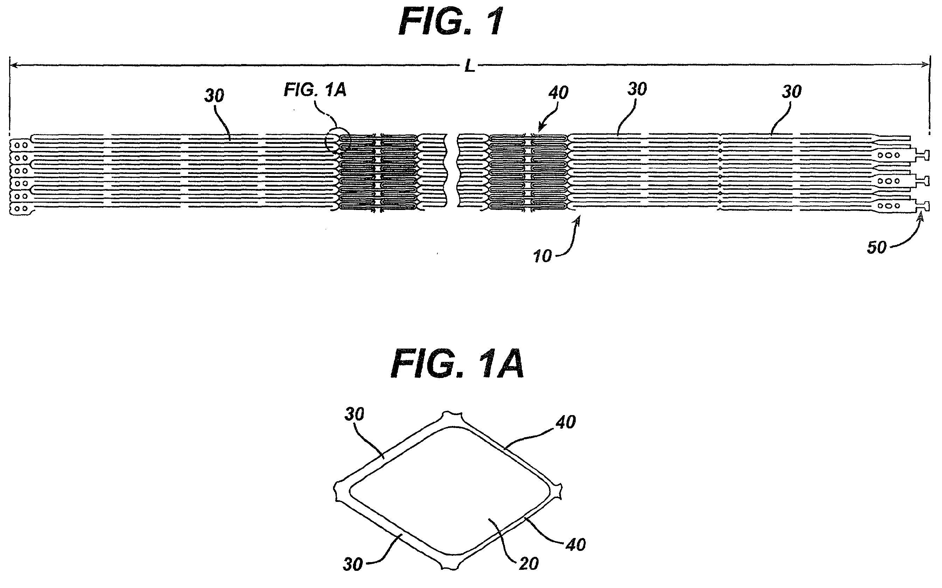

Embodiments of the present invention will now be described by way of example with reference to the accompanying drawings, in which
Figs. 1, 1A, 2, and 3 are schematic views of various embodiments of the present invention.

In terms of background, the three-dimensional, cylindrically symmetric, stent is frequently discussed in terms of a two-dimensional framework, in which the longitudinal direction of the cylinder lies along the horizontal and the circumferential axis lies along the vertical. In expansion, the stent strut changes from its longitudinal (horizontal), as-cut, position, approaching a circumferential (vertical) position. The strut will take on an angled position. This angle is usually lower than 45 degrees (from horizontal) and lower than 90 degrees with respect to the adjacent strut. Angles greater than 45 degrees from horizontal indicate a hyper-expanded state.

The term "recapturable" implies that the device has been fully deployed and the term "re-sheathable" means that the sheath or graft cover can slide back over the device, when it may not have been totally deployed. The term "foreshortening" refers to the reduction in overall device length caused by the horizontal (longitudinal) element rotating towards the vertical. That is, the horizontal element rotates out of the horizontal plane as it bends and the stent opens. The closer it gets to "vertical", the shorter the overall device becomes. This is true because fewer of the device elements (presumably half) are expanded so severely. The others are closer to horizontal, so the overall device loses less of its length.

Referring to the drawings, Figures 1 and 1A show a stent 10 design incorporating diamonds 20 with one side comprised of stiffer struts 30 than the other side, said sides alternating along the longitudinal direction of the stent. The bending of such a stent causes the more compliant struts 40 to buckle along the inside radius of curvature, allowing the stent to flex. Attachment tabs 50 may be incorporated to apply graft material over the stent 10.

An important characteristic of this embodiment is the ability for the stent matrix to be continuous along its length L. The word "continuous" implies that there are no loose corners that would get snagged if one attempted to resheath. Thus, the sheath is fully re-constrainable (or re-capturable or re-sheathable, assuming the device hasn't been completely released). Flexible designs, which can be re-sheathed, are not common. Often, large open spaces are required to accomplish this goal. With the design of the present invention, it is possible to have outward force continuously along the perimeter of the device, which works well against the vessel, and to hold open graft material (or resisting against another adjacent stent).

In "continuous but flexible" stents of the art, there are large spaces around the perimeter so that a zig-zag or diamond pattern must reduce down to "flexible" connection bars (with typical results) or must use multiple reduction steps to go from say, 16 to 8, or from 4 to 2 repeat patterns to attain flexibility. The objective in the art is to have a recapturable device, or at least a re-sheathable one that may be partially deployed during the procedure, and then pulled back in for removal or re-positioning.

The embodiment of the present invention gives advantages over the prior art. Flexible stent designs generally incorporate interrupted or articulated structures such as in the case of SMART stent, or flexible undulating connectors as in the case of the BX velocity stent. Flexibility provided by interrupted or articulated structures have potential disadvantages in terms of recapturability. The present embodiment provides flexibility through an unstable, buckling diamond, thus providing flexibility with fewer drawbacks as to recapturability or delivery profile. Furthermore, the present embodiment flexes by collapsing the inside radius of curvature, without significant stretching of the outside radius. Such a feature is particularly well suited to graft support structures because most grafts also bend by wrinkling or folding the "inside radius" rather than by stretching the "outside radius".

This embodiment can be made in several modifications. The stiffer pair of struts can be made stiffer through a variety of methods, used either alone or in combination. These methods include, but are not limited to, varying the strut length, varying the strut width, varying the strut thickness, varying the angle between the strut and the longitudinal axis, and varying the strut shape. In a preferred embodiment, the stiffer segments are longer and the short diamonds are thinner and more flexible. The collapsing of the short diamond sections under compression (on the inner diameter in bending) allows the design to perform well in bending when covered by graft material.

In another preferred embodiment, the flexible pair of struts is most effectively made flexible by shortening them to the extent that they are at an angle of 90 degrees or greater with respect to each other when the stent is at its operating expansion level. The thinning of these struts to substantially thinner than the adjacent rigid struts also improves flexibility.

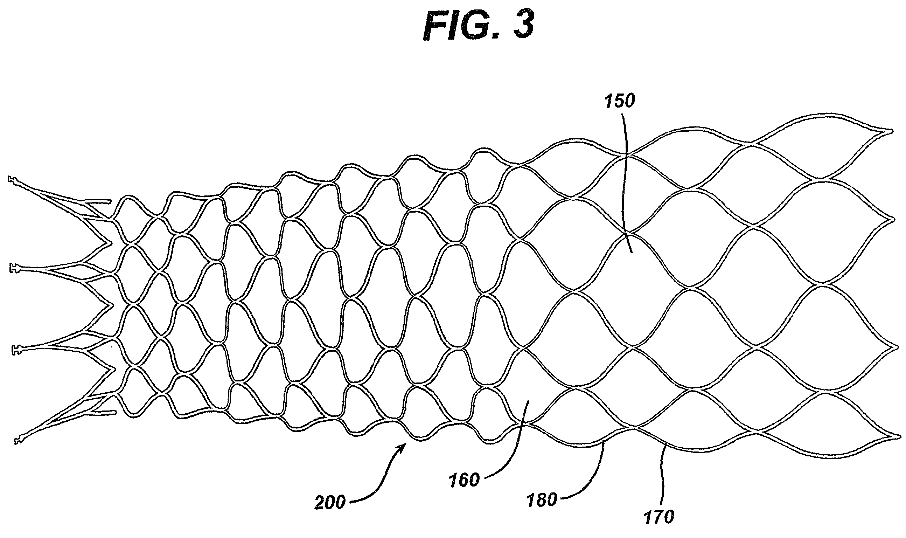

In another preferred embodiment (Figure 3), stents may be formed from a continuous matrix of over-expanded, thin strut diamonds. In this stent 200, the interspersing of diamonds 150, 160 using alternating thin and thick struts 170, 180 respectively allows for a strong yet flexible structure with a "given" direction of bending.

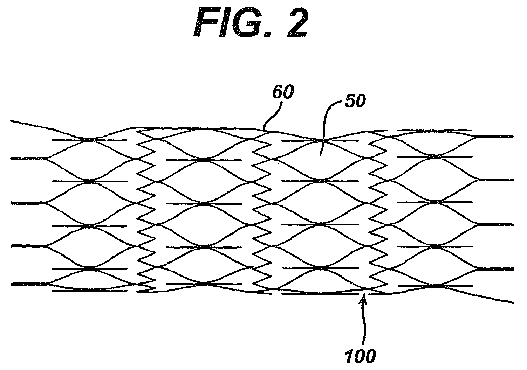

In another preferred embodiment (Figure 2) a stent 100 comprises diamond patterns 50 are alternated with flexible patterns 60. In such a design, the flexible segments immediately adjacent to the rigid struts can flex the most. The other flexible segments resist bending until much more bend and/or force is applied.

Both of the preceding embodiments create a flexible tube stent. The design with the alternating flexible segments within each diamond can perform differently. The flexible segments each bend to the point of buckling in on itself, and this buckling is consistent along the length, which maximizes the bend over a given length of stent.

Any of these embodiments of the present invention describe a self-expanding AAA leg or stent device which comprises a diamond pattern wherein the expanded shape may be extremely oversized. This creates an "inverted2 diamond pattern (a given diamond is longer circumferentially than it is long in the longitudinal direction down the cylinder axis of the stent). This creates a strengthened structure that will resist flattening from external loads (as, for example, in a kissing configuration). Such a structure allows for recapturing because it is not articulated with unusual flexibility compared to typical recapturable designs. Such a pattern can be used selectively within the expanding AAA leg or stent device or can be used down the entire length of the device. Foreshortening may exist, but it can be controlled by accurate placement of the stent by intravascular ultrasound (IVUS).

In embodiments of the present invention, the patterns are used in devices which fold over other structures (as in the case of AAA legs) which require high resistance to flattening. Kissing stent procedures, which are recommended for the AAA, require similar properties.

The present invention discloses oversized diamond patterned legs for AAA devices to create strong, crush resistant devices which are recapturable and flexible. The present invention discloses the use of NiTi self-expandable material to shape a stent so that the diamonds are longer circumferentially than they are longitudinally down the stent. This design makes diamond flexure more stable in longitudinal compression than that of the hoop (circumferential) direction, which promotes flexibility.

In a specific design alternative, one takes advantage of combining a hyperexpanded (over 90 degrees) column of zig-zags, alternating with either a not hyperexpanded or just denser column of zig-zags. This embodiment exhibits a fairly smooth bending arc. This is particularly useful for the large diameter stents, even up to 35 mm in diameter.

## Claims

1. A stent of a plurality of sides and of design comprising diamonds wherein one side comprises stiffer struts than an opposite side.

2. The strut of claim 1 wherein the stiffer segments are longer.

3. The strut of claim 1 wherein in there are long and short diamonds wherein the short diamonds are thinner and more flexible.

4. A stent of a plurality of sides, with one side comprising stiffer struts than the other sides, wherein the more compliant struts buckle along the inside radius of curvature, thereby causing the stent to flex.

5. The stent of claim 4 wherein the stiffer struts are made by a method selected from the group consisting of varying strut length, varying strut width, varying strut thickness, varying the angle between the strut and the longitudinal axis, and varying the strut shape.

6. A stent of a diamond-shaped design wherein there is an inverted diamond pattern, such that a diamond is longer circumferentially than longitudinally.

7. A stent comprising a hyperexpanded column of zig zags alternating with a non-hyperexpanded column of zig zags.

8. A stent comprising a strut which is expanded beyond 45 degrees and which favours bending in the axial direction over bending in the circumferential direction.
